Europäisches Patentamt

European Patent Office

Office européen des brevets

(19)

(11) Publication number: **0 341 290 B1**

## EUROPEAN PATENT SPECIFICATION

(12)

(45) Date of publication of patent specification: **13.07.94**

(51) Int. Cl.5: **C07K 7/00**, C07K 13/00, A61K 37/02, G01N 33/68, //C12P21/00

(21) Application number: **89900066.5**

(22) Date of filing: **18.11.88**

(86) International application number: **PCT/GB88/01018**

(87) International publication number: **WO 89/04834 (01.06.89 89/12)**

(54) IMMUNOGLOBULIN E COMPETITOR.

(30) Priority: **19.11.87 GB 8727045**

(43) Date of publication of application: **15.11.89 Bulletin 89/46**

(45) Publication of the grant of the patent: **13.07.94 Bulletin 94/28**

(84) Designated Contracting States: **AT BE CH DE FR GB IT LI LU NL SE**

(56) References cited:
EP-A- 0 102 634      EP-A- 0 205 405
EP-A- 0 254 249      EP-A- 0 257 114
EP-A- 0 263 655      EP-A- 0 269 455

CHEMICAL ABSTRACT, vol. 107, 1987, Columbus, OH (US); H.L. SPIEGELBERG et al., p. 596, no. 234420s&NUM;

CHEMICAL ABSTRACTS, vol. 109, 1988, Columbus, OH (US); N. NIO et al., p. 525, no. 90892p&NUM;

(73) Proprietor: **3i RESEARCH EXPLOITATION LIMITED**
**91 Waterloo Road**
**London SE1 8XP(GB)**

(72) Inventor: **GOULD, Hannah, Jane**
**48 Great Percy Street**
**London WC1L XOR(GB)**
Inventor: **HELM, Birgit, Anna**
**22 Alderton Hill**
**Loughton Essex IG10 3JB(GB)**
Inventor: **MARSH, Philip, John, Henry, Benedict**
**79a High Street**
**Deptford London SE8 5AA(GB)**

(74) Representative: **Eyles, Christopher Thomas et al**
**W.P. THOMPSON & CO.**
**Celcon House**
**289-293 High Holborn**
**London WC1V 7HU (GB)**

## Description

This invention relates to a competitor or analogue for human Immunoglobulin E (IgE). More particularly, the invention is concerned with a polypeptide which competes with IgE for low-affinity receptor sites.

Our co-pending International Patent Application No. PCT/GB87/00466 (International Patent Publication No. WO88/00204 published 14th January 1988) describes and claims a polypeptide competitor for IgE which has a monomeric chain of seventy-six amino acids from the second and third domains of the human IgE sequence, corresponding to amino acid residues 291 to 366 of the full IgE heavy chain sequence reported by Bennich (Progress in Immunology II, Vol.I, July 1974, pp 49-58 and Int, Arch. Allergy App. Immunol. 53, 459). This polypeptide binds to high-affinity Fc receptors for IgE (FcER$_1$) which exist particularly on mast cells and basophils, thereby inhibiting the biological responses, such as exocytosis or degranulation, which take place when antigen specific IgE binds to and crosslinks such receptor sites in the presence of antigen.

In the human immune system, the principal role of IgE is believed to be to provide immunity to parasites. It also, however, mediates Type I hypersensitivity which is an allergic response leading to the manifestation of such symptoms as hay fever and asthma. Briefly, the mechanism of the allergic response is that on encountering a normally innocuous antigen such as pollen, synthesis of antigen-specific IgE by B-cells is initiated. The antigen-specific IgE then binds to mast cell receptor sites via its Fc region and thereafter any further encounter with the antigen triggers degranulation of the mast cells releasing mediators, principally histamine, resulting in the acute inflammatory symptoms typical of Type I hypersensitivity.

Structurally, IgE, in common with the other immunoglobulins, comprises two heavy and two light chains, the epsilon heavy chain having five domains, a variable domain VH and constant domains CH1 to CH4. The molecular weight of IgE is in the region of 188,000 of which the heavy chain accounts for about 72,500, representing a sequence of approximately 550 amino acid residues.

It has been reported (Nature, vol.315, 1985, No.6020, pp 577-578) that a peptide sequence of 330 amino-acids corresponding to amino acid residues 218 to 547 (in accordance with the numbering given by Bennich, Progress in Immunology II, Vol I, July 1974, pp 49-58) of the epsilon heavy chain of IgE has an inhibitory effect on the release of mediators from human mast cells. The numbering is erroneously assigned in that paper in Nature; the more correct numbering would be 207 to 537. The 330 amino-acid sequence exists as a dimer consisting of two chains of amino-acids, each of 330 amino-acids in length, linked by disulphide bonds.

United States Patents Nos. 4 171 299 and 4 161 522 disclose that an oligopeptide containing from three to ten amino acids in a sequence selected from a portion of amino acids 255 to 527 of the Bennich nomenclature (see reference above) of the Fc region of human IgE will block Fc receptors of mast cells thus inhibiting degranulation and release of mediators such as histamine. The most active of these oligopeptides is identified as the pentapeptide Asp-Ser-Asp-Pro-Arg (called HEPP: Human Immunoglobulin E Polypeptide) derived from the amino acid sequence 320 to 324 of the IgE heavy chain. In native IgE amino acids 322 is asparagine, but it is suggested in these Patents that substitution of asparagine by aspartic acid leads to substantial enhancement of the blocking activity.

In the Patents mentioned above the full sequence which is attributed to Bennich (Progress in Immunology II, Vol I, July 1974, pp 49-58) is quoted and shows aspartic acid at locations 322. However, Bennich himself later asserts (Int. Arch. Allergy Appl. Immunol. 53, 459) that asparagine resides at that location. Bennich also reports that neither of the peptides Asp-Ser-Asp-Pro-Arg nor Asp-Ser-Asn-Pro-Arg has any blocking activity. Determination of the gene sequence has shown that amino acids 322 is asparagine and not aspartic acid. In European Patent Publication No. 0102634 asparagine and not aspartic acid is correctly quoted at the equivalent location.

Further, it is also reported that the specific activity of HEPP is low requiring excessively large doses for any significant physiological effect.

It is known that IgE epsilon chain fragments may be synthesized in Escherichia coli by cloning and expression of the DNA sequences coding for the appropriate domains of the IgE chain (Eur. J. Immunol. 1985, 15: 966-969 and Proc. Natl. Acad. Sc. USA, vol.81, 1984, 2955-2959).

Native IgE binds also to low-affinity receptors (FcER$_2$) on cells such as lymphocytes, eosinophils, monocytes and platelets to activate the effector functions of these cells, for example, IgE-dependent parasite-killing by eosinophils. FcER$_2$ receptors on B lymphocytes have also been implicated in the transduction of growth signals and in B cell activation. FcER$_1$ and FcER$_2$ are structurally unrelated proteins and can be expected to recognize different sites on the Fc region of IgE.

An object of the present invention is to provide a polypeptide which binds to the type 2 low-affinity Fc receptors (FcER$_2$).

According to the present invention there is provided a polypeptide IgE competitor exhibiting specificity for Immunoglobin E low affinity receptor sites comprising a polypeptide having the sequence of amino acid residues 330 to 429 of of the Bennich enumeration, shown in Table I below.

## TABLE I

330  A-Leu-Ser-Arg-Pro-Ser-Pro-Phe-Asp-Leu-

339  Phe-Ile-Arg-Lys-Ser-Pro-Thr-Ile-Thr-Cys-

349  Leu-Val-Val-Asp-Leu-Ala-Pro-Ser-Lys-Gly-

359  Thr-Val-Asn-Leu-Thr-Trp-Ser-Arg-Ala-Ser-

369  Gly-Lys-Pro-Val-Asn-His-Ser-Thr-Arg-Lys-

379  Glu-Glu-Lys-Gln-Arg-Asn-Gly-Thr-Leu-Thr-

389  Val-Thr-Ser-Thr-Leu-Pro-Val-Gly-Thr-Arg-

399  Asp-Trp-Ile-Glu-Gly-Glu-Thr-Tyr-Gln-Cys-

409  Arg-Val-Thr-His-Pro-His-Leu-Pro-Arg-Ala-

419  Leu-Met-Arg-Ser-Thr-Thr-Lys-Thr-Ser-Gly-

429  Pro-B.

The group A in Table I is a hydrogen atom or an amino acid sequence which may include a chain initiating amino acid sequence and which includes at most a part only of the natural sequence of amino acid residues of the heavy chain of Immunoglobulin E, said part of said natural sequence forming a part only of the sequence of amino acid residues 291 to 329 of the Bennich enumeration and B is the sequence of amino acid residues numbered 430 to 537 according to the Bennich enumeration.

A may be an amino acid sequence which corresponds to a part only of the natural amino acid sequence of the heavy chain of Immunoglobulin E extending from residues 329 at the C-terminal end of the group A back up to and including residues 291 using the Bennich enumeration. A may be or include an inert polypeptide sequence, preferably an inert oligopeptide sequence, that does not interfere with the ability of the polypeptide to compete for the Immunoglobulin E low affinity receptor sites.

B corresponds to a part of the IgE heavy chain sequence commencing at residue 430 extending as far as residue 537, using the Bennich enumeration. It will usually be preferred that, when A represents or includes a chain initiating amino acid sequence, such a sequence contains no more than about 20 amino acid residues, and normally no more than about 10 such residues, e.g. 4 or 5.

In one preferred form of polypeptide competitor according to the invention A represents a hydrogen atom or a chain initiating amino acid sequence X, such as Met-Asp-Pro-Arg- (the corresponding nucleotide sequence for which is ATG GAT CCG CGC) and B represents the sequence of amino acid residues 430 to

3

537 of the Bennich enumeration set out in Table II below.

TABLE II

```
430    -Arg-Ala-Ala-Pro-Glu-Val-Tyr-Ala-Phe-
        CGT GCT GCC CCG GAA GTC TAT GCG TTT

439     Ala-Thr-Pro-Glu-Trp-Pro-Gly-Ser-Arg-Asp-
        GCG ACG CCG GAG TGG CCG GGG AGC CGG GAC

449     Lys-Arg-Thr-Leu-Ala-Cys-Leu-Ile-Gln-Asn-
        AAG CGC ACC CTC GCC TGC CTG ATC CAG AAC

459     Phe-Met-Pro-Glu-Asp-Ile-Ser-Val-Gln-Trp-
        TTC ATG CCT GAG GAC ATC TCG GTC CAG TGG

469     Leu-His-Asn-Glu-Val-Gln-Leu-Pro-Asp-Ala-
        CTG CAC AAC GAG GTG CAG CTC CCC GAC GCC

479     Arg-His-Ser-Thr-Thr-Gln-Pro-Arg-Lys-Thr-
        CGC CAC AGC ACG ACG CAG CCC CGC AAG ACC

489     Lys-Gln-Ser-Gly-Phe-Phe-Val-Phe-Ser-Arg-
        AAG GCC TCC GGC TTC TTC GTC TTC AGC CGC

499     Leu-Gln-Val-Thr-Arg-Ala-Glu-Trp-Glu-Gln-
        CTG GAG GTC ACC AGG GCC GAA TGG GAG CAG

509     Lys-Asp-Glu-Phe-Ile-Cys-Arg-Ala-Val-His-
        AAA GAT GAG TTC ATC TGC CGT GCA GTG CAT

519     Glu-Ala-Ala-Ser-Pro-Ser-Gln-Thr-Val-Gln-
        GAG GCA GCG AGC CCC TCA CAG ACC GTC CAG

529     Arg-Ala-Val-Ser-Val-Asn-Pro-Gly-Lys-[Stop]
        CGA GCG GTG TCT GTA AAT CCC GGT AAA TGA.
```

The invention further encompasses a polypeptide competitor as set out in Table I herein wherein A represents the sequence of amino acid residues 291 to 329 of the Bennich enumeration set out in table III below.

4

## TABLE III

291        Gln-Lys-His-Trp-Leu-Ser-Asp-Arg-Thr-Tyr-

301        Thr-Cys-Gln-Val-Thr-Tyr-Gln-Gly-His-Thr-

311        Phe-Glu-Asp-Ser-Thr-Lys-Lys-Cys-Ala-Asp-

321        Ser-Asn-Pro-Arg-Gly-Val-Ser-Ala-Tyr-.

The invention provides in particular a competitor for Immunoglobulin E low affinity receptor sites comprising a polypeptide having the sequence of amino acid residues 330 to 537 of the Bennich enumeration, shown in Table IV below.

Further the invention provides a DNA having the nucleotide sequence also shown in Table IV below.

## TABLE IV

330        X-Leu-Ser-Arg-Pro-Ser-Pro-Phe-Asp-Leu-
           CTA AGC CGG CCC AGC CCG TTC GAC CTG

339        Phe-Ile-Arg-Lys-Ser-Pro-Thr-Ile-Thr-Cys-
           TTC ATC CGC AAG TCG CCC ACG ATC ACC TGT

349        Leu-Val-Val-Asp-Leu-Ala-Pro-Ser-Lys-Gly-
           CTG GTC GTC GAC CTG GCA CCC AGC AAG GGG

359        Thr-Val-Asn-Leu-Thr-Trp-Ser-Arg-Ala-Ser-
           ACC GTG AAC CTG ACC TGG TCC CGC GCC AGT

369        Gly-Lys-Pro-Val-Asn-His-Ser-Thr-Arg-Lys-
           GCG AAG CTT GTG AAC CAC TCC ACC AGA AAG

5

## Table IV continued

```
379   Glu-Glu-Lys-Gln-Arg-Asn-Gly-Thr-Leu-Thr-
      GAG GAG AAG CAG CGC AAT GGC ACG TTA ACC

389   Val-Thr-Ser-Thr-Leu-Pro-Val-Gly-Thr-Arg-
      GTC ACG TCC ACC CTG CCG GTG GGC ACC CGA

399   Asp-Trp-Ile-Glu-Gly-Glu-Thr-Tyr-Gln-Cys-
      GAC TGG ATC GAG GGG GAC ACC TAC CAG TGC

409   Arg-Val-Thr-His-Pro-His-Leu-Pro-Arg-Ala-
      AGG GTG ACC CAC CCC CAC CTG CCC AGG GCC

419   Leu-Met-Arg-Ser-Thr-Thr-Lys-Thr-Ser-Gly-
      CTC ATG CGG TCC ACG ACC AAG ACC AGC GCC

429   Pro-Arg-Ala-Ala-Pro-Glu-Val-Tyr-Ala-Phe-
      CCG CGT GCT GCC CCG GAA GTC TAT GCG TTT

439   Ala-Thr-Pro-Glu-Trp-Pro-Gly-Ser-Arg-Asp-
      GCG ACG CCG GAG TGG CCG GGG AGC CGG GAC

449   Lys-Arg-Thr-Leu-Ala-Cys-Leu-Ile-Gln-Asn-
      AGG CGC ACC CTC GCC TGC CTG ATC CAG AAC

459   Phe-Met-Pro-Glu-Asp-Ile-Ser-Val-Gln-Trp-
      TTC ATG CCT GAG GAC ATC TCG GTC CAG TGG

469   Leu-His-Asn-Glu-Val-Gln-Leu-Pro-Asp-Ala-
      CTG CAC AAC GAG GTG CAG CTC CCC GAC GCC

479   Arg-His-Ser-Thr-Thr-Gln-Pro-Arg-Lys-Thr-
      CGC CAC AGC ACG ACG CAG CCC CGC AAG ACC
```

## Table IV continued

489      Lys-Gln-Ser-Gly-Phe-Phe-Val-Phe-Ser-Arg-
             AAG GCC TCC GGC TTC TTC GTC TTC AGC CGC

499      Leu-Gln-Val-Thr-Arg-Ala-Glu-Trp-Glu-Gln-
             CTG GAG GTC ACC AGG GCC GAA TGG GAG CAG

509      Lys-Asp-Glu-Phe-Ile-Cys-Arg-Ala-Val-His-
             AAA GAT GAG TTC ATC TGC CGT GCA GTG CAT

519      Glu-Ala-Ala-Ser-Pro-Ser-Gln-Thr-Val-Gln-
             GAG GCA GCG AGC CCC TCA CAG ACC GTC CAG

529      Arg-Ala-Val-Ser-Val-Asn-Pro-Gly-Lys-[Stop]
             CGA GCG GTG TCT GTA AAT CCC GGT AAA   TGA

The group X in Table IV is a hydrogen atom or a chain initiating amino acid sequence, and, in the specific method of preparation which will be described below the group X is Met-Asp-Pro-Arg-, the corresponding nucleotide sequence being ATG GAT CCG CGC.

The invention further extends to polypeptide sequences of the types set out in Tables I and IV above (as well as to those set out in Table I as modified by Table II or III terminated at one or both of the N- and C-terminal ends by respective inert oligopeptide sequences initiating and/or terminating the chain).

Dimeric forms of the polypeptide competitors of the invention are of particular interest.

Also included within the scope of the invention are fragments of the polypeptides of the invention, including dimeric forms thereof, having competitive properties which are the same as or similar to those of the sequences defined above in Tables I and IV or in Table I as modified by Table II or Table III.

The invention also provides a host/vector system containing the nucleotide sequence which encodes the polypeptide of sequence 330 to 537.

The expression vector may conveniently be Escherichia coli N4830, and a culture of same harbouring a plasmid (designated as pE3-4) which encodes the peptide of sequence 330 to 537 has been deposited with the National Collection of Type Cultures in London on 18th November 1987 under the Accession Number NCTC 12162.

The invention also provides a method of preparing the polypeptide 330 to 537 comprising culturing the said host organism and isolating the peptide from the culture.

This invention also includes a pharmaceutical preparation in which the active principle is a polypeptide competitor for Immunoglobulin E low affinity receptor sites of the type defined above.

The preparation may also include a pharmaceutical carrier permitting administration of the polypeptide competitor in an appropriate manner, for example intranasally.

The polypeptide competitor of the invention may also be covalently linked to or associated with other therapeutic or diagnostic agents or other molecules with the effect that the polypeptide acts to target the therapeutic or diagnostic agent to cells bearing IgE low affinity receptors.

The invention further contemplates use of the polypeptide competitors of the invention, their dimeric forms and biologically active fragments thereof in a binding assay and for inclusion in a diagnostic kit.

Thus, it has now been discovered that the polypeptide 340-547 of this invention has the ability to bind to the low affinity IgE receptors on cells. An advantage accruing from the invention is that the ability to distinguish between the high and low affinity binding sites of IgE enables separate administration of these effector molecules as may be medically indicated. IgE is associated with allergic reaction known as Type I immediate hypersensitivity and its value as an antagonist is discussed in our co-pending patent application identified above.

The reaction of IgE with mast cells not only leads to immediate hypersensitivity, but is directly responsible for setting up the conditions for delayed hypersensitivity, which is initiated by the release of chemical compounds from mast cells in the initial immune response. Delayed hypersensitivity involves an array of other cell types, such as platelets, macrophages and eosinophils, which are attracted to and cause an inflammatory response at the site of foreign body or initial tissue insult.

When these cells bind via the low affinity receptors to IgE and antigen, cytotoxic oxygen radicals are released which destroy cells in the local vicinity, including foreign cells if present. Thus, the polypeptide 340-547 may he considered for use as an antagonist of IgE to block or diminish the delayed hypersensitivity reaction associated with the natural IgE binding.

The invention will now be described by way of example.

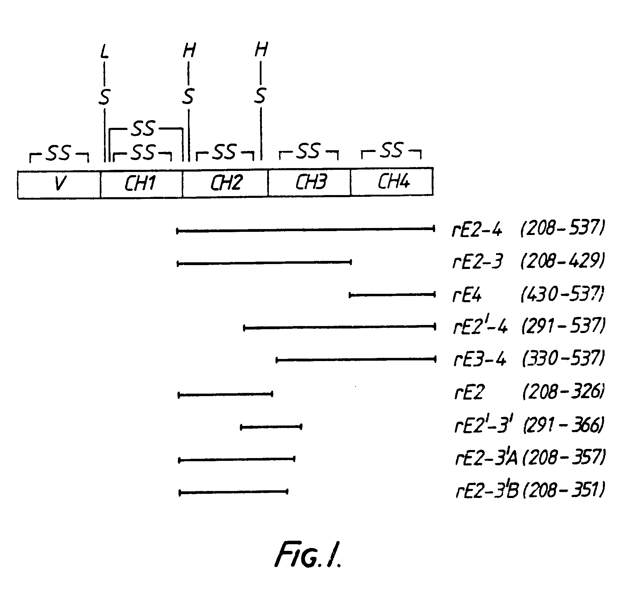

Figure 1 shows the covalent structure of the human epsilon chain of IgE, indicating the positions of the intra-chain disulphide (S-S) bonds and inter-chain disulphide (S-S, S-L), and the boundaries of the five structural domains (VH, CH1-4), corresponding to exons in the genomic DNA, between the N-terminus (left) to the C-terminus (right). The recombinant peptides are described as follows: rE2-4 contains Asp 208-Lys 537 (Ce2-4 with seven amino acids from CH1); rE2-3 contains Asp 208-Pro 429 (CH2 and CH3 with seven amino acids from CH1 and one from CH4); rE4 contains Arg 430-Lys 537; rE2'-4 contains Gln 291-Lys 537 (the C-terminal end of CH2 from the indicated position between the two cysteines that form the inter-chain disulphide bond, CH3 and CH4); rE3-4 contains Leu 330-Lys 537 (all but nine residues at the N-terminus of CH3 and CH4); rE2 contains Asp 208-Val 326 (CH2 with seven amino acids from CH1 and six from CH3); rE2'-3' contains Gln 291-Arg 366 (the C-terminal part of CH2 and the N-terminal part of CH3 from the indicated positions between the cysteines that form intra-chain disulphide bonds in these domains).

Method

The genetic constructs used for expression in E. coli to yield the above peptides are described as follows: plasmids pE2-4 encoding rE2-4 and plasmid pE4 encoding rE4 contain epsilon cDNA fragments bounded by Hind III sites and direct the synthesis of short N-terminal fusions containing the first seven amino acids of the Trp E sequence. The epsilon sequence in pE4 was inserted after addition of an 8-mer Hind III linker (New England Biolabs) to codon 429, the position of an FnuD II site. Plasmid pE2 is a derivative of pE2-4 modified by a 12-mer Nhe I linker (New England Biolabs), which provides translation stop signals. This linker was ligated only to codon 327 in the epsilon sequence after cleavage of the cloned cDNA by Hae II and removal of the 3' overhang with T4 polymerase. Plasmid pE2-3 comprises an epsilon cDNA fragment subcloned into ptac-85 and directs the expression of a non-fusion polypeptide. A translation termination signal (P.L. Biochemicals) was placed immediately downstream of the CH3 sequence by ligating a synthetic oligonucleotide to codon 429, after cleavage of the epsilon sequence at that position with FnuD II. Plasmid pE2'-4 encoding rE2'-4' contains an epsilon gene fragment, which, after cleavage with Sac I (cutting at codon 287) and mild exonuclease treatment with Bal 31, was inserted into the expression vector pASI . The plasmid pE2'- 3' encoding rE2'-3' was made by insertion of a translation terminator into pE2'-4 at the Sma I restriction site located at codon 366.

The rE3-4 sequence was synthesised in E. coli under control of the lambda PL promoter in pE3-4, a recombinant derivative of pASI (Rosenberg et.al., 1983, Meths. Enzymol. 101, 123-138). The gene fragment in pE3-4 was tailored for insertion into the BamHI site of pASI after cleavage of the epsilon DNA with HaeII, mild exonuclease treatment and addition of a 12-mer BamHI linker. The DNA sequence around the BamHI site was determined; from this the deduced amino acid sequence showed that the expressed epsilon sequence started at codon 330. Additional residues introduced in the manipulation are:

```
ATG GAT CCG CGC ....CTA
Met-Asp-Pro-Arg ....Leu-
```

(residue 330 of epsilon sequence)

In E. coli N4830 the epsilon gene fragment in pE3-4 can be expressed by heat induction. To achieve this, growth of the strain to A600 = 0.8 at 30 °C (non-inducing) is followed by incubation at 42 °C (inducing).

The site on human IgE involved in the binding to the low affinity (FcER$_2$) sites on the FcER$_2$- positive human B cell line RPMI 8866 was probed using cloned fragments of human IgE generated in E. coli from the deletion mutants of the gene encoding the CH2, CH3 and CH4 domains of the epsilon (ND) heavy chain. Preliminary indirect and direct immunofluorescence binding assays were carried out on the various fragments and the results are given in Table V below which is an indication of the ability of each peptide to bind to the high affinity receptor.

TABLE V

| Summary of binding of recombinant IgE peptides to human high-affinity (FcR$_1$) and low-affinity (FcR$_2$) receptors. | | | | | | | | |
|---|---|---|---|---|---|---|---|---|
| Peptide | Amino Acids | Heavy Chain Domains | | | | | Binding | |
| | | VH1 | CH1 | CH2 | CH3 | CH4 | FcER1 | FcER2 |
| IgE | 1-537 | + | + | + | + | + | + | + |
| rE2-4 | 208-537 | - | - | + | + | + | + | + |
| rE2'-4 | 291-537 | - | - | part | + | + | + | + |
| rE2-3 | 208-429 | - | - | + | + | - | + | - |
| rE2'-3' | 291-366 | - | - | part | part | - | + | - |
| rE4 | 430-537 | - | - | - | - | + | - | - |
| rE2 | 208-326 | - | - | + | - | - | - | - |
| rE3-4 | 330-537 | - | - | - | + | + | - | + |

These results indicate that the binding of human IgE to FcER$_2$ requires the presence of the third and fourth domains of the heavy chain, as given by the polypeptide of the present invention and that, of our peptides tested, only the peptide rE3-4 binds to the low affinity receptors to the exclusion of high affinity binding.

Further indirect immunofluorescence studies confirmed that the myeloma protein IgE (PS), rE2-4, rE2'-4 and rE3-4 bound to ≧ 90% of FcER$_2$ positive RPMI 8866 B cells. However, this further work showed that, by contrast, there was no detectable binding of rE2-3, rE2'-3', rE4 and rE2. The binding of the rE peptides was specific, since it could be inhibited by preincubation of the cells with two anti-FcER$_2$ monoclonal antibodies (mAb 135 and anti-BLAST-2), but not by control IgGI of unrelated specificity. Moreover, it was found that there was no binding to the FcER$_2$ negative cell lines Jurkat and Raji.

Binding ability of rE peptides

RPMI 8866 cells were incubated with different concentrations of rE peptides and indirect immunofluorescence was performed.

For indirect immunofluorescence the results of which are summarised in Table VI, 0.5 x 10$^6$ RPMI 8866 cells (>99% FcER$_2$ positive) in staining buffer (RPMI 1640 - 2.5% fetal bovine serum, containing 0.01% azide) were incubated with various concentrations (0.1-200 μg/ml) of purified rE fragments or native IgE(PS) for 40 minutes at 4°C. After washing, the cells were incubated for 30 minutes at 4°C with the appropriate fluorescein isothiocyanate (FITC)-conjugated anti-Fc mAB or with an affinity-purified goat anti-human IgE antibody (10 μg/ml). After extensive washing, the percentage of cells binding IgE or the rE fragments was evaluated by a FACScan (Becton Dickinson, Mountain View, CA). Table VI shows the absolute percentage of positive cells (mean ±S.D. of results obtained in 9 experiments).

TABLE VI

| Binding of recombinant IgE peptides to RPMI 8866 B cells by indirect immunofluorescence | | |
|---|---|---|
| Ligand | Amino acid sequence | % positive cells |
| IgE(PS) | Glp I-Lys 537 | 91 ± 2% |
| rE2-4 | Asp 208-Lys 537 | 92 ± 2% |
| rE2'-4 | Gln 291-Lys 537 | 91 ± 2% |
| rE3-4 | Leu 330-Lys 537 | 90 ± 3% |
| rE2-3 | Asp 208-Pro 429 | 3 ± 1% |
| rE2'-3' | Gln 291-Arg 366 | 2 ± 1% |
| rE2 | Asp 208-Val 326 | 2 ± 1% |
| rE4 | Arg 430-Lys 537 | 3 ± 2% |

The data of Table VI indicate that the $FcER_2$ binding site is contained in the rE3-4 peptide (Leu 330 - Lys 537), and does not require the CH2 domain.

For the tests using anti-$FcER_2$ monoclonal antibodies, 0.5 x $10^6$ RPMI 8866 cells in staining buffer were preincubated with medium, anti-$FcER_2$ mAbs (mAb 135 or anti-BLAST-2 mAb), 1 $\mu$g/ml, or a control IgGI murine mAb (anti-HLA-DP, 2.5 $\mu$g/ml) for 60 minutes at 4°C. After washing, the cells were incubated for 40 minutes at 4°C with IgE(PS) (1 $\mu$g/ml) or rE3-4 (200 $\mu$g/ml), followed by mAb RPI-FITC. Table VII shows the absolute percentage of positive cells (mean ± S.D. of results obtained in 3 experiments).

TABLE VII

| Anti-$FcER_2$/CD23 mAbs inhibit the binding of rE3-4 to RPMI 8866 cells | | |
|---|---|---|
| Inhibitor | Ligand | |
| | IgE | rE3-4 |
| Nil | 88±2 | 89±3 |
| mAb 135 | 2±1 | 5±2 |
| anti-BLAST-2 | 3±1 | 4±2 |
| control mouse IgGI | 74±2 | 76±5 |

In these experiments the mAb 135 used was as described by E. Rector et al., Immunology, 55, 481-487 (1985), while the anti-BLAST-2 mAb was as described by C. Kintner et al, Nature, 294, 458-460 (1981).

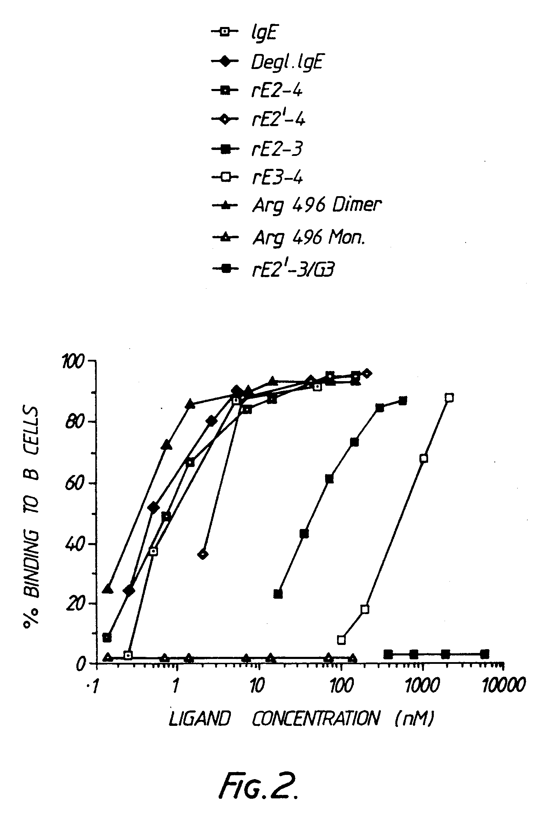

To compare the relative activities of different epsilon-chain fragments and mutant sequences, binding has been measured as a function of peptide concentration. Binding profiles are shown in Figure 2 and the results are summarised in Table VIII. rE2'-3/G3 is a chimaeric recombinant which was constructed using mouse gamma-2b cDNA from pHG201, described by S. Roberts et al, Protein Engineering, 1, 59-65 (1986). The mouse CH3 sequence was derived by partial SacI digestion of pHG201 to yield a DNA fragment encoding amino acids 336-437 (EU index). This fragment was inserted downstream of codon 429 in pE2'-4 replacing the C epsilon-4 sequence with the Cgamma-2b CH3 domain. The recombinant peptide reacted with both epsilon and gamma-2b antisera.

TABLE VIII

| Binding ability of rE peptides | |
|---|---|
| Ligand | Molarity required for 50% binding to B cells |
| IgE | $9.0 \times 10^{-10}$ |
| Deglycosylated IgE | $4.8 \times 10^{-10}$ |
| rE2-4 | $7.2 \times 10^{-10}$ |
| rE2'-4 | $2.6 \times 10^{-9}$ |
| rE3-4 | $5.8 \times 10^{-7}$ |
| rE2-4 Arg 496 (dimer) | $1.6 \times 10^{-10}$ |
| rE2'-3/G3 | $4.7 \times 10^{-8}$ |

For deglycosylation, IgE (PS) (200 $\mu$g/ml) in 0.55 M sodium phosphate, pH 8.6, was incubated with N-glycosydase F (N-Glycanase, Genzyme Corporation, Boston, MA), 15 U/ml, at 37°C overnight, and subsequently absorbed with an excess of Lentil Lectin Sepharose 4 B (Sigma Chemical Company, St. Louis, MO) at 4°C on rotation. (Sepharose is a trademark). Analysis of the preparation by SDS-PAGE and periodic acid-Schiff (PAS) staining of the gel showed that complete deglycosylation of IgE had occurred. IgE concentration in the deglycosylated sample was assessed by radioimmunoassay.

The point substitutions in rE2-4 were carried out by oligonucleotide-directed site specific mutagenesis using double-stranded plasmid DNA in accordance with the procedures described by Y. Morinaga et al, Biotechnology, July, 636-639 (1984).

The recombinant Fc(rE2-4) is highly active, indeed perceptibly more so than myeloma IgE (PS). The elevated affinity is presumably due to the absence of carbohydrate, since our results showed that enzymatic deglycosylation of IgE (PS) increased its activity. rE3-4, which lacks CH2 and nine amino acids from CH3, displayed a much lower activity than rE2-4. A truncated peptide, rE2'-4, retaining the C-terminal thirty amino acids of CH2, by contrast, was almost as active as the full Fc sequence. For physiological binding affinity, therefore, all three Fc domains appear to be necessary.

The structural elements of Fc necessary for binding were defined. The Fc regions of all classes of antibodies are thought to have a number of common features. In particular, they contain two heavy chains, non-covalently linked in their C-terminal domains (e.g. CH4 or C-gamma 3), separated by carbohydrate in the middle domain (CH3 or C-gamma 2), and covalently linked by one or more disulphide bonds in the N-terminal domain (CH2 in IgE, corresponding to the hinge region of IgG). The inter-chain linkages in CH2 and CH4 thus generate a tertiary structure, which may be required for receptor recognition. To determine whether this defines the active state, the activity of monomeric chains has been examined.

All rE chain fragments that contain the CH4 domain can form dimers in solution. All three Fc domains are found to be necessary, however, for the formation of disulphide-linked dimers, suggesting that the non-covalent association in CH4 is required to place the thiols in register in CH2, so that disulphide bond formation can occur. If the association of two CH4 domains nucleates dimer formation, it is conjectured that the formation of dimers could be inhibited by preventing this association. The IgE model suggests that the two Phe 496 residues in CH4 are in van der Waals contact. Thus the replacement of Phe 496 by a charged residue should oppose the formation of dimers. It has been found that the mutant gene expression product, rE2-4 (Arg 496), remains monomeric, as judged by gel electrophoresis. After elimination of a trace of dimer (< 1%) by HPLC, we found the product to be completely inactive in binding to B cells. Strikingly, the dimer, recovered in a separate fraction, was more active than the unmutated Fc. Phe 496 is unlikely to form part of the recognition sequence, since it is buried in the CH4 domain. It is concluded that the dimerisation of chains is essential for FcER$_2$ binding.

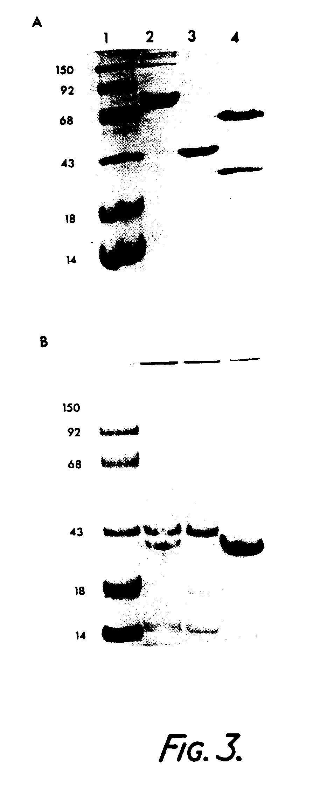

Figure 3 illustrates the results obtained upon polyacrylamide gel electrophoresis of affinity-purified rE peptides. In Figure 3 A designates the results obtained with peptides analyzed in a 20% SDS-urea gel under non-reducing conditions, while B shows the results obtained with peptides analyzed in a 15% SDS-urea gel under reducing conditions. The order of sample application was: lane (1) standard proteins, lane (2) rE2-4, lane (3) rE2-4 (Arg 496), lane (4) rE2'-3/G3. The peptides were isolated from E coli and affinity-purified.

Since CH4 is required for dimerisation and dimerisation for activity, the possibility that part of the binding site lies in CH4 could not be excluded by simply deleting this domain. The monomeric state of rE2-3, for example, is sufficient to account for its inactivity in the binding assay summarized in Table VI above. To define the role of CH4 sequences in FcER$_2$ binding, an investigation was made of the behaviour of the chimaeric immunoglobulin fragment, rE2'-3/G3, in which the C-terminal domain of rE2'-4 is replaced by that of a mouse gamma-2b chain. As expected from the homology between IgE and IgG, the chimaeric chains form disulphide-linked dimers in high yield, as can be seen from Figure 3 which shows the results obtained with rE peptides analysed in SDS- urea gels under non-reducing and reducing conditions. The dimers displayed a level of activity within an order of magnitude of that of their non-chimaeric counterpart as can be appreciated by inspection of Figure 2 which illustrates the results obtained upon polyacrylamide gel electrophoresis of affinity purified rE peptides. Since it was found also that mouse IgG2b did not bind to the B cells, it is concluded that the $FcER_2$-specific binding site is located in CH3.

To map the $FcER_2$ binding site on the CH3 domain more precisely, there were used monoclonal antibodies (mAbs) against epitopes in the Fc region and their efficiency in inhibiting the binding of $^{125}I$-labelled IgE to B cells was measured. The various epitopes recognised by the antibodies were mapped to the rE peptides by Western blotting, and the locations of peptides in the sequence were related to their positions in the model. The results are summarised in Table IX.

TABLE IX

| Anti-human Fc mAbs: specificity and inhibition of $^{125}I$-IgE binding to $FcER_2$ | | |
|---|---|---|
| mAb | epitope location (amino acids) | % inhibition of $^{125}I$-IgE binding |
| DC | CH2 (Asn 207-Gln 291) | none |
| AS 7.12 | CH2 (Asn 207-Gln 291) | none |
| BS 17 | CH2 (Gln 291-Thr 305) | 78 |
| RP 3 | CH2 (Asp 297-Thr 305) | 77 |
| IC 272 | CH2 (Thr 305-Val 326) | 22 |
| Le 27 | CH3 (Leu 330-Val 351) | none |
| RP 1 | CH3 (Leu 330-Val 351) | none |
| IC 27 | CH3 (Lys 357-Val 360) | 88 |
| IC 173 | CH4 (Arg 430-Lys 537) | none |

The specificity of the anti-Fc mAbs was deduced by their pattern of reactivity with the rE fragments, rE fragments containing amino acid residues Asp 208-Thr 305 and a synthetic peptide comprising amino acid residues Asp 297-Val 360 in enzyme-linked immunosorbent assay, Western blot and dot immunoassay. Purified human myeloma IgE(PS) was iodinated by the chloramine-T method (specific activity: 8000 cpm/ng). To test the ability of the anti-Fc mAbs to inhibit the binding of IgE to $FcER_2$, $^{125}I$-IgE (15 ng in 50 μl) in PBS-0.5% BSA was mixed with a 10, 100, 1000 M excess of anti-Fc mAbs for 1 hour at 37°C, and then added to $1 \times 10^6$ RPMI 8866 cells in 0.1 ml. After incubation for 2 hours at 4°C, the cells were spun through serum and the cell-bound radioactivity was counted. Maximal binding was determined by incubating the cells with $^{125}I$-IgE in the presence of medium alone. Table IX shows the inhibition obtained with a 10 M excess of mAb.

Three of the nine monoclonal antibodies strongly inhibited the IgE-receptor interaction. The epitopes for mAb BS 17 and RP 3 lie in the C-terminal region of CH2 (between Gln 291 and Thr 305) and that for the third, IC 27, is at the N-terminal end of CH3 (comprising Lys 357-Val 360). The three sites are located within or near to the cleft between the CH2 and CH3 domains in the model of Fc. A fourth mAb, IC 272, which binds in an intervening sequence (Thr 305-Val 326) was only weakly inhibitory. It is envisaged that this mAb binds to the loop separating the two beta-strands that line the cleft between the CH2 and CH3 domains on the CH2 side. This would afford an explanation for its rather weak inhibitory effect. Two other mAbs, RP 1 and Le 27, which bind within a second intervening sequence (Leu 330-Val 351), had no inhibitory effect. The whole of this peptide segment points away from the cleft. Three other mAbs, DC, AS 7.12 and IC 173, which bind to epitopes outside the CH2-CH3 junction region, in CH2 and CH4, did not inhibit receptor binding. Taken together with the results of the fragment binding assay, these data suggest that $FcER_2$ binds in the vicinity of Asp 352-Val 360. Since CH2 does not contribute sequences to the binding site, inhibition by anti-CH2 mAbs can result only from steric hindrance.

The results discussed above show that the $FcER_2$ binding site on human IgE lies in the N-terminal region of CH3, close to CH2 in the 3D structure of Fc. However, the CH4 and CH2 domains determine the level of activity through their effects on the structure of CH3. In particular, it is found that both CH3 domains are required for activity; CH2 and CH4 serve only to generate a dimer. The native structure in CH2 does not appear to be important per se, since the truncated CH2 domain in rE2'-4 suffices for nearly full activity, and rE3-4 is also active. That in CH4 may also be dispensed with, once covalent bonds are formed, since the substitution of Phe 496 by arginine did not impair activity in the dimer.

The observation that the chimaeric peptide, rE2'-3/G3, displayed significantly less than full activity in binding to the B cell FcER$_2$ requires comment. It is suggested that the C-terminal domain in native IgE may interfere with the binding of IgE to FcER$_2$, and that CH4 constitutes a smaller obstacle to binding than C-gamma-3. It was found that rE2-4 (Arg 496) is more active as a dimer than rE2-4; this might be due to the presence of unpaired CH4 domains, which, owing to their segmental flexibility and rotational freedom, might interfere to a lesser extent than the paired domain. rE4, which appears to have a native structure by the criterion of dimerisation, failed to bind to the FcER$_2$ on the B cell. It is therefore unlikely that the CH4 domain makes a positive contribution to the interaction of IgE with FcER$_2$.

The data do not reveal whether both C-epsilon domains bind to a single receptor or whether one, or possibly two, FcER$_2$ molecules bind to the separate CH3 domains in a dimer. The first (2:1) model of binding seems unlikely, since the two CH3 domains are far apart, being separated by protein and carbohydrate. Existing evidence is inconclusive. It has been shown that mAb IC 27 can bind to an IgE, immobilised on its receptor on a B cell. This could imply that the FcER$_2$ molecule binds to only one of the CH3 domains of IgE, leaving the second free to interact with the mAb (if it is assumed that the mAb and the receptor compete for the same site). Other data, however, suggest that two receptors may bind to the CH3 domains on a single IgE molecule, for a bivalent monoclonal antibody against murine FcER$_2$ binds to the same number of sites as does IgE, while its Fab' fragment binds to twice this number. In theory, "monogamous bivalency", as the 2:2 binding mode has been termed, may offer a large gain in affinity, via. $K_a$ dimer $\geq$ ($K_a$ monomer)$^2$. If the observed $K_a$ of $10^7$ M$^{-1}$ arises from comparable contributions at both sites, then the binding free energy developed by a single-site interaction may be below the threshold of detection used in the assay described herein. There is still uncertainty about the stoichiometry of the interaction which translates into a question of specificity, namely whether or not the receptor recognises a single CH3 domain in a conformation induced by dimerisation, or whether or not the dimer serves only to generate higher affinity through interaction at two sites.

The FcER$_2$ binding site is distinct from the FcER$_1$ binding site in human IgE: FcER$_1$, but not FcER$_2$, binds to rE2-3 and rE2'-3', whereas FcER$_2$, but not FcER$_1$, binds to rE3-4. This indicates that some part of the sequence between Gln 291 and Leu 330 is required for FcER$_1$, but not for FcER$_2$ binding. The sites may overlap in CH3, in the region between Val 352 and Lys 357, which forms the C-terminal boundary of the FcER$_1$ site, but the FcER$_2$ site may extend further towards the C-terminal side, or indeed the sites may be totally separate. Higher-resolution mapping is required to establish the N-terminal boundary of the FcER$_2$ binding site and thus determine the extent, if any, of common sequence. The two receptors also exhibit a different mode of binding, since FcER$_1$ binds to monomeric and dimeric chains, wherein FcER$_2$ fails to bind to monomers. The monocyte receptor binding site on IgGl and the T and B cell receptor binding site on IgM have been mapped, respectively, to the C-gamma-2 and C-mu-3 domains, homologous to CH3. It is of interest that Fc-mu-R, like FcER$_2$, fails to bind to monomeric chains.

FcER$_1$ and FcER$_2$ are unrelated proteins; FcER$_1$ belongs to the immunoglobulin superfamily, in common with all other immunoglobulin receptors so far described. FcER$_2$ is unique, in that it is homologous to the asialoglycoprotein receptor. It is therefore surprising that FcER$_2$ binds to rE2-4, and to enzymatically deglycosylated IgE, even more strongly than to native IgE. High affinity binding of IgG to Fc-gamma-RI, on the contrary, is reported to be dependent on the presence of carbohydrate. It is noted that there is a glycosylation site at Asn 361 in the epsilon chain near the putative region of the FcER$_2$ binding site. This carbohydrate substituent is clearly not a part of the binding site, but it is found only in epsilon-heavy chains and might be well placed to modulate FcER$_2$ binding activity.

## Claims

**1.** A polypeptide IgE competitor exhibiting specificity for Immunoglobulin E low affinity receptor sites comprising a polypeptide having the sequence of amino acid residues, numbered 330 to 537 according to the Bennich enumeration, of the formula:

13

330     A-Leu-Ser-Arg-Pro-Ser-Pro-Phe-Asp-Leu-

339     Phe-Ile-Arg-Lys-Ser-Pro-Thr-Ile-Thr-Cys-

349     Leu-Val-Val-Asp-Leu-Ala-Pro-Ser-Lys-Gly-

359     Thr-Val-Asn-Leu-Thr-Trp-Ser-Arg-Ala-Ser-

369     Gly-Lys-Pro-Val-Asn-His-Ser-Thr-Arg-Lys-

379     Glu-Glu-Lys-Gln-Arg-Asn-Gly-Thr-Leu-Thr-

389     Val-Thr-Ser-Thr-Leu-Pro-Val-Gly-Thr-Arg-

399     Asp-Trp-Ile-Glu-Gly-Glu-Thr-Tyr-Gln-Cys-

409     Arg-Val-Thr-His-Pro-His-Leu-Pro-Arg-Ala-

419     Leu-Met-Arg-Ser-Thr-Thr-Lys-Thr-Ser-Gly-

429     Pro-Arg-Ala-Ala-Pro-Glu-Val-Tyr-Ala-Phe-

439     Ala-Thr-Pro-Glu-Trp-Pro-Gly-Ser-Arg-Asp-

449     Lys-Arg-Thr-Leu-Ala-Cys-Leu-Ile-Gln-Asn-

459     Phe-Met-Pro-Glu-Asp-Ile-Ser-Val-Gln-Trp-

```
469        Leu-His-Asn-Glu-Val-Gln-Leu-Pro-Asp-Ala-

479        Arg-His-Ser-Thr-Thr-Gln-Pro-Arg-Lys-Thr-

489        Lys-Gln-Ser-Gly-Phe-Phe-Val-Phe-Ser-Arg-

499        Leu-Gln-Val-Thr-Arg-Ala-Glu-Trp-Glu-Gln-

509        Lys-Asp-Glu-Phe-Ile-Cys-Arg-Ala-Val-His-

519        Glu-Ala-Ala-Ser-Pro-Ser-Gln-Thr-Val-Gln-

529        Arg-Ala-Val-Ser-Val-Asn-Pro-Gly-Lys-[Stop],
```

wherein the group A is a hydrogen atom or an amino acid sequence which may include a chain initiating amino acid sequence and in which said amino acid sequence includes at most a part only of the natural sequence of amino acid residues of the heavy chain of Immunoglobulin E, said part of said natural sequence forming a part only of the sequence of amino acid residues, numbered 291 to 329 according to the Bennich enumeration, of the following formula:

```
291        Gln-Lys-His-Trp-Leu-Ser-Asp-Arg-Thr-Tyr-

301        Thr-Cys-Gln-Val-Thr-Tyr-Gln-Gly-His-Thr-

311        Phe-Glu-Asp-Ser-Thr-Lys-Lys-Cys-Ala-Asp-

321        Ser-Asn-Pro-Arg-Gly-Val-Ser-Ala-Tyr-
```

2. A polypeptide competitor according to claim 1, wherein A represents a part of the sequence of amino acid residues, numbered 291 to 329 according to the Bennich enumeration, of the following formula:

```
291        Gln-Lys-His-Trp-Leu-Ser-Asp-Arg-Thr-Tyr-

301        Thr-Cys-Gln-Val-Thr-Tyr-Gln-Gly-His-Thr-

311        Phe-Glu-Asp-Ser-Thr-Lys-Lys-Cys-Ala-Asp-

321        Ser-Asn-Pro-Arg-Gly-Val-Ser-Ala-Tyr-.
```

3. A polypeptide competitor according to claim 1, wherein A represents the amino acid sequence Met-Asp-Pro-Arg-.

4. A polypeptide competitor for Immunoglobulin E low affinity receptor sites comprising a polypeptide having the sequence of amino acid residues, numbered 330 to 537 according to the Bennich enumeration, of the formula:

```
330      X-Leu-Ser-Arg-Pro-Ser-Pro-Phe-Asp-Leu-

339      Phe-Ile-Arg-Lys-Ser-Pro-Thr-Ile-Thr-Cys-

349      Leu-Val-Val-Asp-Leu-Ala-Pro-Ser-Lys-Gly-

359      Thr-Val-Asn-Leu-Thr-Trp-Ser-Arg-Ala-Ser-

369      Gly-Lys-Pro-Val-Asn-His-Ser-Thr-Arg-Lys-

379      Glu-Glu-Lys-Gln-Arg-Asn-Gly-Thr-Leu-Thr-

389      Val-Thr-Ser-Thr-Leu-Pro-Val-Gly-Thr-Arg-

399      Asp-Trp-Ile-Glu-Gly-Glu-Thr-Tyr-Gln-Cys-
```

409  Arg-Val-Thr-His-Pro-His-Leu-Pro-Arg-Ala-

419  Leu-Met-Arg-Ser-Thr-Thr-Lys-Thr-Ser-Gly-

429  Pro-Arg-Ala-Ala-Pro-Glu-Val-Tyr-Ala-Phe-

439  Ala-Thr-Pro-Glu-Trp-Pro-Gly-Ser-Arg-Asp-

449  Lys-Arg-Thr-Leu-Ala-Cys-Leu-Ile-Gln-Asn-

459  Phe-Met-Pro-Glu-Asp-Ile-Ser-Val-Gln-Trp-

469  Leu-His-Asn-Glu-Val-Gln-Leu-Pro-Asp-Ala-

479  Arg-His-Ser-Thr-Thr-Gln-Pro-Arg-Lys-Thr-

489  Lys-Gln-Ser-Gly-Phe-Phe-Val-Phe-Ser-Arg-

499  Leu-Gln-Val-Thr-Arg-Ala-Glu-Trp-Glu-Gln-

509  Lys-Asp-Glu-Phe-Ile-Cys-Arg-Ala-Val-His-

519  Glu-Ala-Ala-Ser-Pro-Ser-Gln-Thr-Val-Gln-

529  Arg-Ala-Val-Ser-Val-Asn-Pro-Gly-Lys-[Stop]

wherein X is a hydrogen atom or a chain initiating amino acid sequence.

5. A polypeptide competitor according to claim 4 wherein X represents Met-Asp-Pro-Arg.

6. A polypeptide competitor according to any one of claims 1 to 5 wherein the specified polypeptide sequence is terminated at at least one end by an inert oligopeptide sequence initiating or terminating the chain.

7. A polypeptide competitor according to any one of claims 1 to 6 wherein the specified polypeptide sequence is terminated both at its N-terminal end and at its C-terminal end by respective inert oligopeptide sequences initiating and terminating the chain.

8. A polypeptide competitor according to claim 4 wherein X is a hydrogen atom.

9. A dimeric form of a polypeptide competitor according to any one of claims 1 to 8.

10. Fragments of a polypeptide competitor according to any one of claims 1 to 8 or a dimeric form thereof according to claim 9 having competitive properties which are the same as or similar to those of the specified polypeptide sequence.

11. A pharmaceutical preparation containing as active principle a polypeptide competitor according to any one of claims 1 to 8, or a dimeric form according to claim 9, or a biologically active fragment thereof as claimed in claim 10 in combination with a pharmaceutical carrier or excipient.

12. A DNA having the nucleotide sequence:

**CTA AGC CGG CCC AGC CCG TTC GAC CTG**

**TTC ATC CGC AAG TCG CCC ACG ATC ACC TGT**

**CTG GTC GTC GAC CTG GCA CCC AGC AAG GGG**

ACC GTG AAC CTG ACC TGG TCC CGC GCC AGT

GCG AAG CTT GTG AAC CAC TCC ACC AGA AAG

GAG GAG AAG CAG CGC AAT GGC ACG TTA ACC

GTC ACG TCC ACC CTG CCG GTG GGC ACC CGA

GAC TGG ATC GAG GGG GAC ACC TAC CAG TGC

AGG GTG ACC CAC CCC CAC CTG CCC AGG GCC

CTC ATG CGG TCC ACG ACC AAG ACC AGC GCC

CCG CGT GCT GCC CCG GAA GTC TAT GCG TTT

GCG ACG CCG GAG TGG CCG GGG AGC CGG GAC

AGG CGC ACC CTC GCC TGC CTG ATC CAG AAC

TTC ATG CCT GAG GAC ATC TCG GTC CAG TGG

CTG CAC AAC GAG GTG CAG CTC CCC GAC GCC

CGC CAC AGC ACG ACG CAG CCC CGC AAG ACC

AAG GCC TCC GGC TTC TTC GTC TTC AGC CGC

CTG GAG GTC ACC AGG GCC GAA TGG GAG CAG

AAA GAT GAG TTC ATC TGC CGT GCA GTG CAT

GAG GCA GCG AGC CCC TCA CAG ACC GTC CAG

CGA GCG GTG TCT GTA AAT CCC GGT AAA TGA

**13.** Fragments of the DNA claimed in claim 12 which, in a host/vector system, express at least one fragment according to claim 10.

**14.** A transformant in which the DNA of the transforming vector contains a nucleotide sequence according to claim 12 or claim 13.

**15.** A vector comprising a DNA segment according to claim 12 or claim 13 said segment being oriented within said vector such that in a host said segment is expressed to produce a polypeptide.

**16.** A vector as claimed in claim 15, comprising the plasmid pE 3-4 deposited in Escherichia coli N4830 with the National Collection of Type Cultures, London on 18th November 1987 under the Accession Number NCTC 12162.

**17.** A host transformed by the vector claimed in claim 16.

**18.** A host/vector as claimed in claim 17 comprising Escherichia coli N4830 harbouring the plasmid pE 3-4 (Accession Number NCTC 12162, deposited with the National Collection of Type Cultures, London on 18th November 1987).

**19.** A method of preparing the polypeptide claimed in claim 4, comprising culturing the host organism claimed in claim 17 and isolating the polypeptide from the culture.

**20.** A method of preparing the polypeptide claimed in claim 8 comprising culturing the host/vector claimed in claim 18, isolating the polypeptide from the culture to obtain the polypeptide claimed in claim 4 and treating same to remove the chain initiating group X.

**21.** A binding assay in which a reagent is used which is a polypeptide competitor according to any one of claims 1 to 8 or a dimeric form according to claim 9 or a biologically active fragment according to claim 10.

**22.** A diagnostic kit including means for conducting an assay and including, for use as a reagent in the said assay, a polypeptide competitor according to any one of claims 1 to 8 or a dimeric form according to claim 9 or a biologically active fragment according to claim 10.

**Patentansprüche**

**1.** Polypeptid-IgE-Kompetitor mit Spezifität für Rezeptorstellen mit niedriger Affinität für Immunglobulin E, aufweisend ein Polypeptid, welches die Aminosäuresequenz der folgenden Formel aufweist, wobei die Aminosäurereste die Nummern 330 bis 537 gemäß der Bennich-Liste tragen:

```
330    A-Leu-Ser-Arg-Pro-Ser-Pro-Phe-Asp-Leu-
339    Phe-Ile-Arg-Lys-Ser-Pro-Thr-Ile-Thr-Cys-
349    Leu-Val-Val-Asp-Leu-Ala-Pro-Ser-Lys-Gly-
359    Thr-Val-Asn-Leu-Thr-Trp-Ser-Arg-Ala-Ser-
369    Gly-Lys-Pro-Val-Asn-His-Ser-Thr-Arg-Lys-
379    Glu-Glu-Lys-Gln-Arg-Asn-Gly-Thr-Leu-Thr-
389    Val-Thr-Ser-Thr-Leu-Pro-Val-Gly-Thr-Arg-
399    Asp-Trp-Ile-Glu-Gly-Glu-Thr-Tyr-Gln-Cys-
409    Arg-Val-Thr-His-Pro-His-Leu-Pro-Arg-Ala-
419    Leu-Met-Arg-Ser-Thr-Thr-Lys-Thr-Ser-Gly-
```

```
429    Pro-Arg-Ala-Ala-Pro-Glu-Val-Tyr-Ala-Phe-
439    Ala-Thr-Pro-Glu-Trp-Pro-Gly-Ser-Arg-Asp-
449    Lys-Arg-Thr-Leu-Ala-Cys-Leu-Ile-Gln-Asn-
459    Phe-Met-Pro-Glu-Asp-Ile-Ser-Val-Gln-Trp-
469    Leu-His-Asn-Glu-Val-Gln-Leu-Pro-Asp-Ala-
479    Arg-His-Ser-Thr-Thr-Gln-Pro-Arg-Lys-Thr-
489    Lys-Gln-Ser-Gly-Phe-Phe-Val-Phe-Ser-Arg-
499    Leu-Gln-Val-Thr-Arg-Ala-Glu-Trp-Glu-Gln-
509    Lys-Asp-Glu-Phe-Ile-Cys-Arg-Ala-Val-His-
519    Glu-Ala-Ala-Ser-Pro-Ser-Gln-Thr-Val-Gln
529    Arg-Ala-Val-Ser-Val-Asn-Pro-Gly-Lys-[Stop],
```

wobei die Gruppe A ein Wasserstoffatom oder eine Aminosäuresequenz bedeutet, die eine ketteninitiierende Aminosäuresequenz einschließen kann, und wobei die Aminosäuresequenz höchstens einen Teil lediglich der natürlichen Aminosäuresequenz der schweren Kette von Immunglobulin E einschließt, wobei der Teil der natürlichen Sequenz einen Teil lediglich der Aminosäuresequenz der folgenden Formel bildet, wobei die Aminosäurereste die Nummern 291 bis 329 gemäß der Bennich-Liste tragen:

```
291    Gln-Lys-His-Trp-Leu-Ser-Asp-Arg-Thr-Tyr-
301    Thr-Cys-Gln-Val-Thr-Tyr-Gln-Gly-His-Thr-
311    Phe-Glu-Asp-Ser-Thr-Lys-Lys-Cys-Ala-Asp-
321    Ser-Asn-Pro-Arg-Gly-Val-Ser-Ala-Tyr-
```

2. Polypeptid-Kompetitor nach Anspruch 1, wobei A einen Teil der Aminosäuresequenz der folgenden Formel darstellt, wobei die Aminosäurereste die Nummern 291 bis 329 gemäß der Bennich-Liste tragen:

```
291    Gln-Lys-His-Trp-Leu-Ser-Asp-Arg-Thr-Tyr-
301    Thr-Cys-Gln-Val-Thr-Tyr-Gln-Gly-His-Thr-
311    Phe-Glu-Asp-Ser-Thr-Lys-Lys-Cys-Ala-Asp-
321    Ser-Asn-Pro-Arg-Gly-Val-Ser-Ala-Tyr-.
```

3. Polypeptid-Kompetitor nach Anspruch 1, wobei A die Aminosäuresequenz Met-Asp-Pro-Arg- darstellt.

4. Polypeptid-Kompetitor für Rezeptorstellen mit niedriger Affinität für Immunglobulin E, aufweisend ein Polypeptid, welches die Aminosäuresequenz der folgenden Formel aufweist, wobei die Aminosäurereste die Nummern 330 bis 537 gemäß der Bennich-Liste tragen:

```
330     X-Leu-Ser-Arg-Pro-Ser-Pro-Phe-Asp-Leu-
339     Phe-Ile-Arg-Lys-Ser-Pro-Thr-Ile-Thr-Cys-
349     Leu-Val-Val-Asp-Leu-Ala-Pro-Ser-Lys-Gly-
359     Thr-Val-Asn-Leu-Thr-Trp-Ser-Arg-Ala-Ser-
369     Gly-Lys-Pro-Val-Asn-His-Ser-Thr-Arg-Lys-
379     Glu-Glu-Lys-Gln-Arg-Asn-Gly-Thr-Leu-Thr-
389     Val-Thr-Ser-Thr-Leu-Pro-Val-Gly-Thr-Arg-
399     Asp-Trp-Ile-Glu-Gly-Glu-Thr-Tyr-Gln-Cys-
409     Arg-Val-Thr-His-Pro-His-Leu-Pro-Arg-Ala-
419     Leu-Met-Arg-Ser-Thr-Thr-Lys-Thr-Ser-Gly-
429     Pro-Arg-Ala-Ala-Pro-Glu-Val-Tyr-Ala-Phe-
439     Ala-Thr-Pro-Glu-Trp-Pro-Gly-Ser-Arg-Asp-
449     Lys-Arg-Thr-Leu-Ala-Cys-Leu-Ile-Gln-Asn-
459     Phe-Met-Pro-Glu-Asp-Ile-Ser-Val-Gln-Trp-
469     Leu-His-Asn-Glu-Val-Gln-Leu-Pro-Asp-Ala-
479     Arg-His-Ser-Thr-Thr-Gln-Pro-Arg-Lys-Thr-
489     Lys-Gln-Ser-Gly-Phe-Phe-Val-Phe-Ser-Arg-
499     Leu-Gln-Val-Thr-Arg-Ala-Glu-Trp-Glu-Gln-
509     Lys-Asp-Glu-Phe-Ile-Cys-Arg-Ala-Val-His-
519     Glu-Ala-Ala-Ser-Pro-Ser-Gln-Thr-Val-Gln
529     Arg-Ala-Val-Ser-Val-Asn-Pro-Gly-Lys-[Stop]
```

wobei X ein Wasserstoffatom oder eine ketteninitiierende Aminosäuresequenz bedeutet.

5.  Polypeptid-Kompetitor nach Anspruch 4, wobei X Met-Asp-Pro-Arg darstellt.

6.  Polypeptid-Kompetitor nach einem der Ansprüche 1 bis 5, wobei die spezifizierte Polypeptidsequenz an mindestens einem Ende von einer inerten Oligopeptidsequenz terminiert wird, die die Kette initiiert oder terminiert.

7.  Polypeptid-Kompetitor nach einem der Ansprüche 1 bis 6, wobei die spezifizierte Polypeptidsequenz sowohl an ihrem N-terminalen Ende als auch an ihrem C-terminalen Ende von jeweiligen inerten Oligopeptidsequenzen terminiert wird, die die Kette initiieren und terminieren.

8.  Polypeptid-Kompetitor nach Anspruch 4, wobei X ein Wasserstoffatom bedeutet.

9.  Dimere Form eines Polypeptid-Kompetitors nach einem der Ansprüche 1 bis 8.

10. Fragmente eines Polypeptid-Kompetitors nach einem der Ansprüche 1 bis 8 oder eine dimere Form davon nach Anspruch 9, aufweisend kompetitive Eigenschaften, die gleich oder ähnlich denen der spezifizierten Polypeptidsequenz sind.

11. Pharmazeutische Zubereitung, enthaltend als Wirkstoff einen Polypeptid-Kompetitor nach einem der Ansprüche 1 bis 8 oder eine dimere Form nach Anspruch 9 oder ein biologisch aktives Fragment davon nach Anspruch 10 in Kombination mit einem pharmazeutischen Träger- oder Vehikelstoff.

**12.** DNA mit der Nucleotidsequenz:

CTA AGC CGG CCC AGC CCG TTC GAC CTG

TTC ATC CGC AAG TCG CCC ACG ATC ACC TGT

CTG GTC GTC GAC CTG GCA CCC AGC AAG GGG

```
ACC GTG AAC CTG ACC TGG TCC CGC GCC AGT

GCG AAG CTT GTG AAC CAC TCC ACC AGA AAG

GAG GAG AAG CAG CGC AAT GGC ACG TTA ACC

GTC ACG TCC ACC CTG CCG GTG GGC ACC CGA

GAC TGG ATC GAG GGG GAC ACC TAC CAG TGC

AGG GTG ACC CAC CCC CAC CTG CCC AGG GCC

CTC ATG CGG TCC ACG ACC AAG ACC AGC GCC

CCG CGT GCT GCC CCG GAA GTC TAT GCG TTT

GCG ACG CCG GAG TGG CCG GGG AGC CGG GAC

AGG CGC ACC CTC GCC TGC CTG ATC CAG AAC

TTC ATG CCT GAG GAC ATC TCG GTC CAG TGG

CTG CAC AAC GAG GTG CAG CTC CCC GAC GCC

CGC CAC AGC ACG ACG CAG CCC CGC AAG ACC

AAG GCC TCC GGC TTC TTC GTC TTC AGC CGC

CTG GAG GTC ACC AGG GCC GAA TGG GAG CAG

AAA GAT GAG TTC ATC TGC CGT GCA GTG CAT

GAG GCA GCG AGC CCC TCA CAG ACC GTC CAG

CGA GCG GTG TCT GTA AAT CCC GGT AAA TGA
```

13. Fragmente der DNA gemäß Anspruch 12, die in einem Wirts/Vektor-System mindestens ein Fragment gemäß Anspruch 10 exprimieren.

**EP 0 341 290 B1**

**14.** Transformant, bei dem die DNA des Transformierungsvektors eine Nucleotidsequenz gemäß Anspruch 12 oder 13 enthält.

**15.** Vektor, aufweisend ein DNA-Segment gemäß Anspruch 12 oder 13, wobei das Segment innerhalb des Vektors so orientiert ist, daß das Segment in einem Wirt zum Erzeugen eines Polypeptids exprimiert wird.

**16.** Vektor nach Anspruch 15, aufweisend das Plasmid pE 3-4, hinterlegt in Escherichia coli N4830 bei der National Collection of Type Cultures, London am 18. November 1987 unter der Eingangsnummer NCTC 12162.

**17.** Wirt, transformiert durch einen Vektor gemäß Anspruch 16.

**18.** Wirt/Vektor nach Anspruch 17, aufweisend Escherichia coli N4830, beherbergend das Plasmid pE 3-4 (Eingangsnummer NCTC 12162, hinterlegt bei der National Collection of Type Cultures, London am 18. November 1987).

**19.** Verfahren zum Herstellen des Polypeptids gemäß Anspruch 4, bei dem man den Wirtsorganismus gemäß Anspruch 17 kultiviert und das Polypeptid aus der Kultur isoliert.

**20.** Verfahren zum Herstellen des Polypeptids gemäß Anspruch 8, bei dem man den Wirt/Vektor gemäß Anspruch 18 kultiviert, das Polypeptid aus der Kultur zum Erhalten des Polypeptids gemäß Anspruch 4 isoliert und dasselbe zum Entfernen der ketteninitiierenden Gruppe X behandelt.

**21.** Bindungs-Assay unter Verwendung eines Reagenzes, welches ein Polypeptid-Kompetitor nach einem der Ansprüche 1 bis 8 oder eine dimere Form nach Anspruch 9 oder ein biologisch aktives Fragment nach Anspruch 10 ist.

**22.** Diagnostische Packung, umfassend Mittel zum Durchführen eines Assays und zur Anwendung als Reagenz in diesem Assay einen Polypeptid-Kompetitor nach einem der Ansprüche 1 bis 8 oder eine dimere Form nach Anspruch 9 oder ein biologisch aktives Fragement nach Anspruch 10.

**Revendications**

**1.** Compétiteur d'IgE polypeptidique présentant une spécificité pour les sites récepteurs de faible affinité de l'immunoglobuline E, comprenant un polypeptide ayant la séquence de résidus d'aminoacides, numérotés de 330 à 537 suivant la numérotation de Bennich, de la formule:

330      A-Leu-Ser-Arg-Pro-Ser-Pro-Phe-Asp-Leu-

339      Phe-Ile-Arg-Lys-Ser-Pro-Thr-Ile-Thr-Cys-

349      Leu-Val-Val-Asp-Leu-Ala-Pro-Ser-Lys-Gly-

359      Thr-Val-Asn-Leu-Thr-Trp-Ser-Arg-Ala-Ser-

369      Gly-Lys-Pro-Val-Asn-His-Ser-Thr-Arg-Lys-

379      Glu-Glu-Lys-Gln-Arg-Asn-Gly-Thr-Leu-Thr-

389      Val-Thr-Ser-Thr-Leu-Pro-Val-Gly-Thr-Arg-

399      Asp-Trp-Ile-Glu-Gly-Glu-Thr-Tyr-Gln-Cys-

409      Arg-Val-Thr-His-Pro-His-Leu-Pro-Arg-Ala-

419      Leu-Met-Arg-Ser-Thr-Thr-Lys-Thr-Ser-Gly-

429      Pro-Arg-Ala-Ala-Pro-Glu-Val-Tyr-Ala-Phe-

439      Ala-Thr-Pro-Glu-Trp-Pro-Gly-Ser-Arg-Asp-

449      Lys-Arg-Thr-Leu-Ala-Cys-Leu-Ile-Gln-Asn-

459      Phe-Met-Pro-Glu-Asp-Ile-Ser-Val-Gln-Trp-

```
469        Leu-His-Asn-Glu-Val-Gln-Leu-Pro-Asp-Ala-

479        Arg-His-Ser-Thr-Thr-Gln-Pro-Arg-Lys-Thr-

489        Lys-Gln-Ser-Gly-Phe-Phe-Val-Phe-Ser-Arg-

499        Leu-Gln-Val-Thr-Arg-Ala-Glu-Trp-Glu-Gln-

509        Lys-Asp-Glu-Phe-Ile-Cys-Arg-Ala-Val-His-

519        Glu-Ala-Ala-Ser-Pro-Ser-Gln-Thr-Val-Gln-

529        Arg-Ala-Val-Ser-Val-Asn-Pro-Gly-Lys-[Stop],
```

dans laquelle le groupe A est un atome d'hydrogène ou une séquence d'aminoacides qui peut inclure une séquence d'aminoacides initiatrice de chaîne et dans lequel ladite séquence d'aminoacides ne comprend au plus qu'une partie de la séquence de résidus d'aminoacides naturelle de la chaîne lourde de l'immunoglobuline E, ladite partie de ladite séquence naturelle ne formant qu'une partie de la séquence de résidus d'aminoacides, numérotés de 291 à 329 selon la numérotation de Bennich, de la formule suivante:

```
291        Gln-Lys-His-Trp-Leu-Ser-Asp-Arg-Thr-Tyr-

301        Thr-Cys-Gln-Val-Thr-Tyr-Gln-Gly-His-Thr-

311        Phe-Glu-Asp-Ser-Thr-Lys-Lys-Cys-Ala-Asp-

321        Ser-Asn-Pro-Arg-Gly-Val-Ser-Ala-Tyr-
```

**2.** Compétiteur polypeptidique selon la revendication 1, dans lequel A représente une partie de la séquence de résidus d'aminoacides, numérotés de 291 à 329 selon la numérotation de Bennich, de la formule suivante:

27

291  Gln-Lys-His-Trp-Leu-Ser-Asp-Arg-Thr-Tyr-

301  Thr-Cys-Gln-Val-Thr-Tyr-Gln-Gly-His-Thr-

311  Phe-Glu-Asp-Ser-Thr-Lys-Lys-Cys-Ala-Asp-

321  **Ser-Asn-Pro-Arg-Gly-Val-Ser-Ala-Tyr-.**

3. Compétiteur polypeptidique selon la revendication 1, dans lequel A représente la séquence d'aminoacides Met-Asp-Pro-Arg.

4. Compétiteur polypeptidique pour les sites récepteurs de faible affinité de l'immunoglobuline E, comprenant un polypeptide ayant la séquence de résidus d'aminoacides, numérotés de 330 à 537 selon la numérotation de Bennich, de formule:

330  X -Leu-Ser-Arg-Pro-Ser-Pro-Phe-Asp-Leu-

339  Phe-Ile-Arg-Lys-Ser-Pro-Thr-Ile-Thr-Cys-

349  Leu-Val-Val-Asp-Leu-Ala-Pro-Ser-Lys-Gly-

359  Thr-Val-Asn-Leu-Thr-Trp-Ser-Arg-Ala-Ser-

369  Gly-Lys-Pro-Val-Asn-His-Ser-Thr-Arg-Lys-

379  Glu-Glu-Lys-Gln-Arg-Asn-Gly-Thr-Leu-Thr-

389  Val-Thr-Ser-Thr-Leu-Pro-Val-Gly-Thr-Arg-

399  Asp-Trp-Ile-Glu-Gly-Glu-Thr-Tyr-Gln-Cys-

409  Arg-Val-Thr-His-Pro-His-Leu-Pro-Arg-Ala-

419  Leu-Met-Arg-Ser-Thr-Thr-Lys-Thr-Ser-Gly-

```
429        Pro-Arg-Ala-Ala-Pro-Glu-Val-Tyr-Ala-Phe-

439        Ala-Thr-Pro-Glu-Trp-Pro-Gly-Ser-Arg-Asp-

449        Lys-Arg-Thr-Leu-Ala-Cys-Leu-Ile-Gln-Asn-

459        Phe-Met-Pro-Glu-Asp-Ile-Ser-Val-Gln-Trp-

469        Leu-His-Asn-Glu-Val-Gln-Leu-Pro-Asp-Ala-

479        Arg-His-Ser-Thr-Thr-Gln-Pro-Arg-Lys-Thr-

489        Lys-Gln-Ser-Gly-Phe-Phe-Val-Phe-Ser-Arg-

499        Leu-Gln-Val-Thr-Arg-Ala-Glu-Trp-Glu-Gln-

509        Lys-Asp-Glu-Phe-Ile-Cys-Arg-Ala-Val-His-

519        Glu-Ala-Ala-Ser-Pro-Ser-Gln-Thr-Val-Gln-

529        Arg-Ala-Val-Ser-Val-Asn-Pro-Gly-Lys-[Stop]
```

dans laquelle X est un atome d'hydrogène ou une séquence d'aminoacides initiatrice de chaîne.

5. Compétiteur polypeptidique selon la revendication 4, dans lequel X représente Met-Asp-Pro-Arg.

6. Compétiteur polypeptidique selon l'une quelconque des revendications 1 à 5, dans lequel la séquence polypeptidique spécifiée est terminée à au moins une extrémité par une séquence oligopeptidique inerte initiant ou terminant la chaîne.

7. Compétiteur polypeptidique selon l'une quelconque des revendications 1 à 6, dans lequel la séquence polypeptidique spécifiée est terminée à la fois à l'extrémité N-terminale et à l'extrémité C-terminale par des séquences oligopeptidiques inertes initiant et terminant respectivement la chaîne.

8. Compétiteur polypeptidique selon la revendication 4, dans lequel X est un atome d'hydrogène.

9. Forme dimère d'un compétiteur polypeptidique selon l'une quelconque des revendications 1 à 8.

10. Fragments d'un compétiteur polypeptidique selon l'une quelconque des revendications 1 à 8 ou d'une forme dimère selon la revendication 9, ayant des propriétés compétitrices qui sont identiques ou similaires à celles de la séquence polypeptidique spécifiée.

11. Composition pharmaceutique contenant comme principe actif un compétiteur polypeptidique selon l'une quelconque des revendications 1 à 8, ou une forme dimère selon la revendication 9, ou un de ses fragments biologiquement actifs selon la revendication 10, en combinaison avec un support ou excipient pharmaceutique.

12. ADN ayant la séquence de nucléotides:

CTA AGC CGG CCC AGC CCG TTC GAC CTG

TTC ATC CGC AAG TCG CCC ACG ATC ACC TGT

CTG GTC GTC GAC CTG GCA CCC AGC AAG GGG

ACC GTG AAC CTG ACC TGG TCC CGC GCC AGT

GCG AAG CTT GTG AAC CAC TCC ACC AGA AAG

GAG GAG AAG CAG CGC AAT GGC ACG TTA ACC

GTC ACG TCC ACC CTG CCG GTG GGC ACC CGA

GAC TGG ATC GAG GGG GAC ACC TAC CAG TGC

AGG GTG ACC CAC CCC CAC CTG CCC AGG GCC

CTC ATG CGG TCC ACG ACC AAG ACC AGC GCC

CCG CGT GCT GCC CCG GAA GTC TAT GCG TTT

EP 0 341 290 B1

```
GCG ACG CCG GAG TGG CCG GGG AGC CGG GAC

AGG CGC ACC CTC GCC TGC CTG ATC CAG AAC

TTC ATG CCT GAG GAC ATC TCG GTC CAG TGG

CTG CAC AAC GAG GTG CAG CTC CCC GAC GCC

CGC CAC AGC ACG ACG CAG CCC CGC AAG ACC

AAG GCC TCC GGC TTC TTC GTC TTC AGC CGC

CTG GAG GTC ACC AGG GCC GAA TGG GAG CAG

AAA GAT GAG TTC ATC TGC CGT GCA GTG CAT

GAG GCA GCG AGC CCC TCA CAG ACC GTC CAG

CGA GCG GTG TCT GTA AAT CCC GGT AAA TGA
```

**13.** Fragments de l'ADN selon la revendication 12 qui, dans un système hôte/vecteur, expriment au moins un fragment selon la revendication 10.

**14.** Cellule transformée dans laquelle l'ADN du vecteur transformant contient une séquence de nucléotides selon la revendication 12 ou la revendication 13.

**15.** Vecteur comprenant un segment d'ADN selon la revendication 12 ou la revendication 13, ledit segment étant orienté dans ledit vecteur de façon que, dans un hôte, ledit segment s'exprime pour produire un polypeptide.

**16.** Vecteur selon la revendication 15, comprenant le plasmide pE 3-4 déposé dans *Escherichia coli* N4830 auprès de la National Collection of Type Cultures, Londres, le 18 novembre 1987, sous le numéro d'accès NCTC 12162.

**17.** Hôte transformé par le vecteur selon la revendication 16.

**18.** Hôte/vecteur selon la revendication 17 comprenant *Escherichia coli* N4830 abritant le plasmide pE 3-4 (numéro d'accès NCTC 12162, déposé auprès de la National Collection of Type Cultures, Londres, le 18 novembre 1987).

**19.** Méthode de préparation du polypeptide selon la revendication 4, comprenant la culture de l'organisme hôte selon la revendication 17 et l'isolement du polypeptide à partir de la culture.

**20.** Méthode de préparation du polypeptide selon la revendication 8, dans laquelle on cultive l'hôte/vecteur selon la revendication 18, on isole le polypeptide à partir de la culture pour obtenir le polypeptide selon

31

la revendication 4, et on le traite pour éliminer le groupe X initiateur de chaîne.

21. Dosage faisant intervenir une liaison (entre deux substances) dans lequel on utilise un réactif qui est un compétiteur polypeptidique selon l'une quelconque des revendications 1 à 8, ou une forme dimère selon la revendication 9 ou un fragment biologiquement actif selon la revendication 10.

22. Trousse de diagnostic comprenant des moyens pour effectuer un dosage et contenant, en tant que réactif à utiliser dans ledit dosage, un compétiteur polypeptidique selon l'une quelconque des revendications 1 à 8 ou une forme dimère selon la revendication 9 ou un fragment biologiquement actif selon la revendication 10.

FIG. 1.

FIG.2.

FIG. 3.